# EUROPEAN PATENT APPLICATION

(11) **EP 4 660 615 A1**
(43) Date of publication of application: **10.12.2025**
(21) Application number: 23919293.3
(22) Date of filing: 09.08.2023
(51) Int. Cl.: G01N 21/78, G01N 27/08, E03D 9/00

(54) **TEST MEMBER FOR URINE TEST, AND TOILET COMPRISING SAME**

(30) Priority: 31.01.2023 CN 202320158607 U
(71) Applicant: Esmart (Xiamen) Technology Co., Ltd., Xiamen, Fujian 361000 (CN)
(72) Inventor: LIU, Zhipeng, Xiamen, Fujian 361000 (CN); LIN, Jian, Xiamen, Fujian 361000 (CN); ZHANG, Zulian, Xiamen, Fujian 361000 (CN); HUANG, Candong, Xiamen, Fujian 361000 (CN); ZHONG, Zhijun, Xiamen, Fujian 361000 (CN)
(74) Representative: Chung, Hoi Kan
(86) International application number: PCT/CN2023/111987
(87) International publication number: WO 2024/159728

(57) **Abstract**

A toilet with a test member for urine test. The toilet comprises a control unit, a toilet body (10), and a test unit provided at the toilet body (10); the test unit comprises a test member (20) and an analysis device (40) provided on a side of the test member (20); the test member (20) comprises a movable support (202), and a test paper (203) is provided on the support (202); in an initial state, the support (202) retracts, and during testing, the support (202) is driven to extend to a preset area; a sensing device (30) is provided at the toilet body (10), the sensing device (30) is used for testing the presence of urine in a bowl, and the control unit controls the test unit according to the test result of the sensing device (30). The sensing device (30) can be used for accurately controlling the reaction time of the test paper (203), thereby improving the testing accuracy of the test paper (203). The analysis device (40) is provided to reduce recovery of the test paper (203), making the test more convenient.

## Description

### TECHNICAL FIELD

The utility model relates to a toilet with a test member for urine test, applied in the field of toilets.

### BACKGROUND

With advancements in technology and growing health awareness, more and more users are purchasing personal health monitoring devices to track their physical condition. As a novel urine testing means, urine test toilets have begun emerging in the public eye.

### SUMMARY

### Technical problem

For example, in a test paper delivery mechanism described in patent CN202220194859.0, test paper falls into a bowl through a paper delivery slide, comes into contact with excrement, and tests corresponding indicators. This process is automated. The test paper delivery mechanism arranged inside a toilet eliminates pipeline cleaning of a cleaning system, and has the advantages of high cleanliness, high hygiene, and convenient use. However, the test paper arranged outside the toilet has poor sealing property. Due to long-term placement, the test paper in the packaging box may get damp to affect test results. Moreover, the reaction time between urine and the test paper should not be too long, otherwise, overreaction will lead to inaccurate test results. Therefore, the urine test toilet needs to accurately test the time when the user starts urinating. For one or more of the above problems, the utility model designs a toilet with a test member for urine test.

### Technical solution:

The utility model provides a toilet with a test member for urine test, which can effectively solve the above problems.

The utility model is implemented as follows:

A toilet with a test member for urine test includes a control unit, a toilet body, and a test unit provided at the toilet, where the test unit includes a test member and an analysis device provided on a side of the test member;
the test member includes a movable support, and a test paper is provided on the support; and
a sensing device is provided at the toilet body, the sensing device is used for testing the presence of urine in a bowl, and the control unit controls the test unit according to the test result of the sensing device.

Preferably, the sensing device tests the conductivity or TDS (Total Dissolved Solids) of a trapped water in the bowl of the toilet body, the turbidity of the trapped water, the liquid level of the trapped water, or whether there is a water column in the bowl; and after the tested conductivity value, TDS value, turbidity value, or liquid level value is greater than a preset value, or after the variation value of the tested conductivity, TDS, turbidity, or liquid level is greater than a preset value, or when a water column is tested, the analysis device analyzes the test paper.

Preferably, in an initial state, the support retracts, and during testing, the support is driven to extend to a preset area; the control unit further includes a trapped water test circuit; the trapped water test circuit controls the sensing device at the beginning of test to test the conductivity, TDS, turbidity, or liquid level of the trapped water; and when the conductivity, TDS, turbidity, or liquid level is less than the preset value, the control unit controls the support to extend to the preset area.

Preferably, the test member further includes an outer shell, the outer shell is provided with a first opening allowing the support to extend and retract relative to the outer shell, and the support is movable between an extended position relative to the outer shell and a retracted position relative to the outer shell.

Preferably, the outer shell further includes a second opening opposite to the first opening, the support is provided with a driving portion at the end close to the second opening, and when the support is at the retracted position, the driving portion is located outside the outer shell.

Preferably, the driving portion includes a rack and a motor, the motor drives the rack, and the rack is connected to the support at the end close to the second opening.

Preferably, a sealing set is provided at two ends of the support; the sealing set includes a first sealing member and a second sealing member; when the support is located at the retracted position, the first sealing member is close to the first opening, the second sealing member is close to the second opening, and the support and the outer shell are sealed by the first sealing member and the second sealing member to form a sealing chamber for accommodating the test paper; and when the support is located at the extended position, the second sealing member is close to the first opening.

Preferably, the analysis device is a color sensor, and during analysis, the support retracts from the second opening for test and analysis.

Preferably, the sensing device is a conductivity sensor, a turbidity sensor, a liquid level sensor, or a radar, and the sensing device is arranged diagonally upward from a horizontal line.

Preferably, the test member is installed on the toilet body; in the initial state, the support retracts; and during testing, the support is driven to extend into the bowl of the toilet body.

The sensing device tests the conductivity or TDS of the trapped liquid in the bowl of the toilet body, the turbidity of the trapped water, the liquid level of the trapped liquid, or whether there is a water column in the bowl; and after the tested conductivity value, TDS value, turbidity value, or liquid level value is greater than a preset value, or after the variation value of the tested conductivity, TDS, turbidity, or liquid level is greater than a preset value, or when a water column is tested, the control unit controls the support to retract, and the analysis device analyzes the test paper.
Beneficial effects are as follows:
(1) The test member includes the support, and the test paper is provided on the support; during testing, the support is driven to extend to the preset area; the sensing device is provided on the toilet body, the sensing device is used for testing the presence of urine in the bowl, and the control unit controls the test unit according to the test result of the sensing device, where the sensing device can be used for accurately controlling the reaction time of the test paper, thereby improving the testing accuracy of the test paper; and the analysis device is provided to reduce recovery of the test paper, making the test more convenient.
(2) The sensing device is provided on the toilet body, and the analysis device is provided on the side of the test member; during sensing test within the toilet body, the test member automatically extends for testing, where the sensing device can be used for accurately controlling the reaction time of the test paper, thereby improving the testing accuracy of the test paper; and the analysis device is provided to reduce recovery of the test paper, making the test more convenient.

### BRIEF DESCRIPTION OF THE DRAWINGS

To illustrate the technical solutions of the embodiments of the utility model more clearly, the accompanying drawings required for use in the embodiments will be briefly introduced below. It should be understood that the following accompanying drawings show only some embodiments of the utility model and should not be regarded as limitations on the scope. For those of ordinary skill in the art, other relevant drawings can also be obtained based on these drawings without any creative efforts.
FIG. 1 is a schematic diagram of a cross-sectional structure provided in an embodiment of the utility model.
FIG. 2 is a schematic diagram of an enlarged structure at FIG. 1-A.
FIG. 3 is a schematic diagram of a cross-sectional structure of a test member provided in an embodiment of the utility model.
FIG. 4 is a schematic structural diagram of a second clamping portion provided in an embodiment of the utility model.
FIG. 5 is a schematic structural diagram of a first clamping portion provided in an embodiment of the utility model.
FIG. 6 is a schematic structural diagram of a driving portion provided in another embodiment.

In the figures:
Toilet body;
101. First clamping portion;
Test member;
201. Outer shell; 201a. First opening; 201b. Second opening; 201c. Slide rail; 202. Support; 202a. First sealing member; 202b. Second sealing member; 202c. Chute; 203. Test paper; 204. Driving portion; 204a. Rack; 204b. Motor; 204c. Cylinder;
Sensing device;
301. Second clamping portion;
40. Analysis device.

### DETAILED DESCRIPTION

To make the objectives, technical solutions, and advantages of the embodiments of the utility model clearer, the technical solutions of the embodiments of the utility model will be described clearly and completely in conjunction with the accompanying drawings therein. Obviously, the described embodiments are some of the embodiments of the utility model, not all of them. Based on the embodiments of the utility model, all other embodiments obtained by those of ordinary skill in the art without any creative effort fall within the scope of protection of the utility model. Therefore, the following detailed descriptions of the embodiments of the utility model provided in the accompanying drawings are not intended to limit the scope of the utility model, but only to represent selected embodiments of the utility model. Based on the embodiments of the utility model, all other embodiments obtained by those of ordinary skill in the art without any creative effort fall within the scope of protection of the utility model.

In the description of the utility model, the terms "first" and "second" are only used for descriptive purposes and should not be understood as indicating or implying relative importance or implicitly indicating the quantity of technical features. Thus, the features defined by "first" or "second" can explicitly or implicitly include one or more of these features. In the description of the utility model, the term "a plurality of" means two or more, unless otherwise clearly and specifically limited.

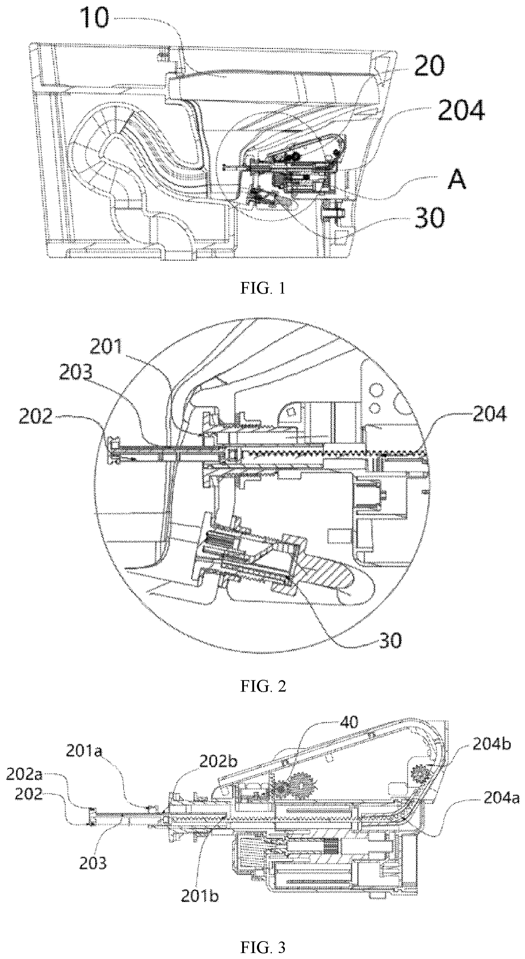

As shown in FIGs. 1-6, a toilet with a test member for urine test includes a control unit, a toilet body 10, and a test unit provided on the toilet body 10. The test unit includes a test member 20 and an analysis device 40 provided on a side of the test member 20.

The test member 20 is provided on a side away from a drain outlet of the toilet body 10, and the test member 20 includes an outer shell 201. In this embodiment, the outer shell 201 includes a first sleeve and a second sleeve, the second sleeve is fitted onto the first sleeve, the first sleeve and the second sleeve are connected by threads, and the second sleeve is provided on the toilet body 10. A movable support 202 is provided in the outer shell 201, and test paper 203 is provided on the support 202. In an initial state, the test paper 203 is sealed in the outer shell 201, and the test paper 203 is in a sealed state. During testing, the support 202 is driven to expose the test paper 203 from the outer shell 201, and the test paper 203 fully contacts urine to complete a sampling process.

A sensing device 30 is provided below the test member 20, the sensing device 30 is used for testing the presence of urine in a bowl, and the control unit controls the test unit according to the test result of the sensing device 30. The control unit further includes a trapped water test circuit, and the trapped water test circuit controls the sensing device 30 to test the conductivity or TDS in the bowl at the beginning of test. When the conductivity or TDS is less than a preset value, the control unit controls the support 202 to extend out of the outer shell 201. The sensing device 30 includes electrodes, and the distance between the test member 20 and the sensing device 30 is 20 cm. The distance of 20 cm between the test member 20 and the sensing device 30 ensures effective testing by the sensing device 30. The sensing device 30 is a conductivity sensor, which includes electrodes. When urine test is activated and in a trapped water evacuating stage, a main control adjusts the conductivity sensor to a trapped water evacuating test mode. In this mode, when the conductivity sensor tests that its electrodes are exposed to air, that is, the conductivity is infinitesimal, normal trapped water evacuation is determined, a trapped water evacuating signal is issued, and then a driving portion 204 can push the test paper out. After the urine test device is activated, in this embodiment, the conductivity sensor continuously tests the conductivity value in the ceramic bowl, where the conductivity value is converted into TDS units. When a variation in conductivity or the conductivity greater than 4000 us/cm is tested, it is considered that the user is urinating. Then, the driving portion 204 pulls the test paper back into the urine test mechanism. The sensing device 30 is provided below the test member 20, so that urine passes through the test paper first and then reaches the conductivity sensor, thereby avoiding retraction of the support 202 before the test paper 203 gets wet to affect test results. The conductivity sensor is connected to a delay device, and a delay recovery time of 5 seconds is set for sufficient sampling after a urine signal is detected. In other embodiments, for precise test results, the delay recovery time may be set to 10 seconds, without excessive restrictions here. The toilet body 10 is provided with a first clamping portion 101, the sensing device 30 is provided with a second clamping portion 301, the first clamping portion 101 is connected to the second clamping portion 301, and the analysis device 40 is provided on the side of the test member 20. During sensing test within the toilet body 10, the test member 20 automatically extends for testing. The analysis processing reduces recovery of the test paper 203, making the test more convenient.

In some embodiments, the sensing device 30 further includes a turbidity sensor. After the tested turbidity value of the trapped water is greater than a set value or the tested turbidity variation value of the trapped water is greater than a set value, the control unit controls the support 202 to retract, and the analysis device 40 analyzes the test paper 203. When the tested turbidity value of the trapped water is less than the preset value, the control unit controls the support 202 to extend to a preset area.

In some embodiments, the sensing device 30 further includes a liquid level sensor. After the tested liquid level value in the trapped water is greater than a set value or the tested variation value of the liquid level of the trapped water is greater than a set value, the control unit controls the support 202 to retract, and the analysis device 40 analyzes the test paper 203. When the tested liquid level value in the trapped water is less than the preset value, the control unit controls the support 202 to extend to the preset area.

In some embodiments, the sensing device 30 further includes a radar. When a water column is tested in the bowl, the control unit controls the support 202 to retract, and the analysis device 40 analyzes the test paper 203.

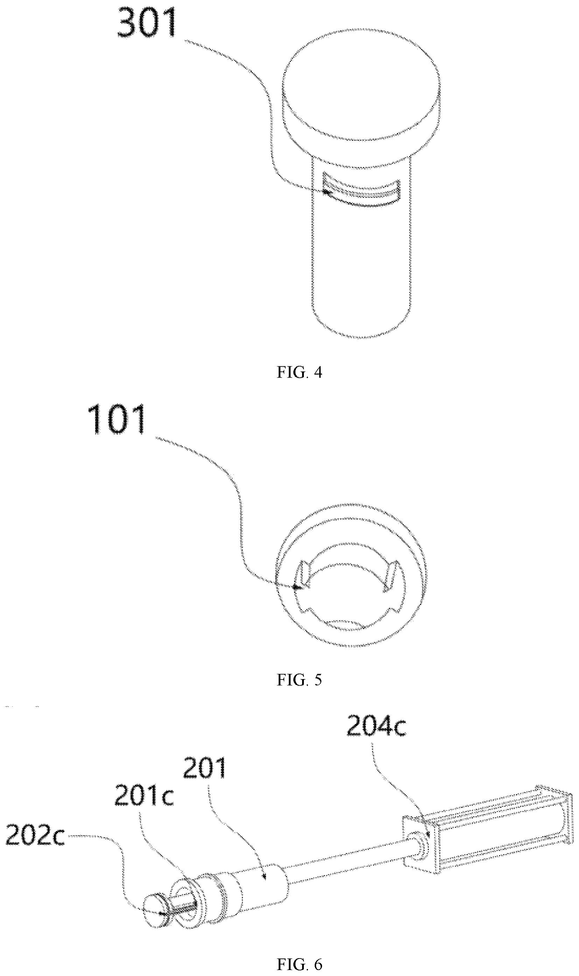

As a further improvement, the outer shell 201 is provided with a first opening 201a allowing the support 202 to extend and retract relative to the outer shell 201, the support 202 is movable between an extended position relative to the outer shell 201 and a retracted position relative to the outer shell 201, the first opening 201a is opposite to the second opening 201b, the support 202 is provided with a driving portion 204 at the end close to the second opening 201b, and when the support is at the retracted position, the driving portion 204 is located outside the outer shell 201. In this embodiment, the driving portion 204 includes a rack 204a connected to the support 202 at the end close to the second opening 201b of the outer shell 201, an arc-shaped rack guide groove is provided on the inner wall of the toilet body 10, the rack 204a is a flexible rack, and the rack 204a slides within the rack guide groove. The arc-shaped rack guide groove is provided to increase the movable range of the rack, facilitating the extension and retraction of the support 202. The rack 204a is driven by a motor 204b to drive a gear to rotate, thereby achieving the extension and retraction functions of the support 202, as shown in FIG. 6. In other embodiments, the driving portion 204 includes a slide rail 201c provided on the outer shell 201 and a chute 202c provided on the support 202 and adapted to the slide rail, the slide rail 201c is slidably connected to the chute 202c, the support 202 is connected to a cylinder 204c, and the cylinder drives the support to extend and retract.

As a further improvement, a sealing set is provided at two ends of the support 202; the sealing set includes a first sealing member 202a and a second sealing member 202b; when the support is located at the retracted position, the first sealing member 202a is close to the first opening 201a, the second sealing member 202b is close to the second opening 201b, and the support 202 and the outer shell 201 are sealed by the first sealing member 202a and the second sealing member 202b to form a sealing chamber for accommodating the test paper 203; and when the support is located at the extended position, the second sealing member 202b is close to the first opening 201a, thereby blocking water from entering the outer shell 201. The outer shell 201 and the sealing set provided on the support 201 package a single piece of disposable test paper 203, thereby effectively avoiding the problem in the prior art that unused test paper placed outside the toilet gets damp and ineffective to affect test results.

As a further improvement, the analysis device 40 is a color sensor. During analysis, the color sensor reads the RGB (Red Green Blue) value of the test paper to obtain a test result.

The first clamping portion 101 is connected to the second clamping portion 301 to facilitate the disassembly of the conductivity sensor for functional calibration and replacement. In one embodiment, the first clamping portion 101 is connected to the second clamping portion 301 by threads, a positioning hole is formed on a bottom side wall at the threaded portion of the sensing device 30, and a nut is installed on the positioning hole. The nut is installed to clamp and fix the sensing device 30 with the ceramic hole, and the sensing device 30 can be rotated and removed, as shown in FIGs. 4 and 5. In another embodiment, a retaining edge extending outward is provided on a side wall of the sensing device 30, and a bearing platform adapted to the retaining edge is provided on the toilet body 10. The second clamping portion 301 is an oblique arc-shaped lug, and the first clamping portion 101 is a groove adapted to the arc-shaped lug. When the retaining edge is borne on the bearing platform, the sensing device 30 is rotated, and the arc-shaped lug is clamped with the groove to complete fixation. The sensing device 30 can be removed by rotating it.

As a further improvement, the sensing device 30 is a conductivity sensor, which is arranged diagonally upward from a horizontal line. In this embodiment, the sensing device 30 is integrated on a nozzle, and a spray outlet of the nozzle needs to be inclined to align with the drain outlet.

Taking the conductivity sensor as an example, the working principle of the utility model is as follows:
1. The conductivity sensor is installed at the ceramic bottom. When urine test is activated and in the trapped water evacuating stage, the main control adjusts the conductivity sensor to the trapped water evacuating test mode. In this mode, when the conductivity sensor tests that its electrodes are exposed to the air, that is, the conductivity is infinitesimal, normal trapped water evacuation is determined, a trapped water evacuating signal is issued, and then the driving portion 204 can push the test paper out. After the urine test device is activated, the conductivity sensor continuously tests the conductivity value in the ceramic bowl. When a variation in conductivity or the conductivity greater than 2000 ppm or 4000 us/cm is tested, it is considered that the user is urinating, and then the driving portion 204 pulls the test paper 203 back into the outer shell 201.
2. After urine is tested, the driving portion 204 pulls the test paper back immediately or after a preset delay time. In this embodiment, the preset delay time is 5 seconds.
3. After being pulled back, the test paper 203 is tested and analyzed by the color sensor. The color sensor reads the RGB value of the test paper to obtain a test result.

Merely preferred embodiments of the utility model are described above and are not intended to limit the utility model. For those skilled in the art, the utility model can be modified and changed in various ways. Any modification, equivalent substitution, improvement, etc. made within the spirit and principles of the utility model shall fall within the scope of protection of the utility model.

## Claims

1. A toilet with a test member for urine test, wherein the toilet comprises a control unit, a toilet body (10), and a test unit provided at the toilet; the test unit comprises a test member (20) and an analysis device (40) provided on a side of the test member (20);
the test member (20) comprises a movable support (202), and a test paper (203) is provided on the support (202); and
a sensing device (30) is provided at the toilet body (10), the sensing device (30) is used for testing the presence of urine in a bowl, and the control unit controls the test unit according to the test result of the sensing device (30).

2. The toilet with the test member for urine test according to claim 1, wherein the sensing device (30) tests the conductivity or TDS of trapped water in the bowl of the toilet body (10), the turbidity of the trapped water, the liquid level of the trapped water, or whether there is a water column in the bowl; and after the tested conductivity value, TDS value, turbidity value, or liquid level value is greater than a preset value, or after the variation value of the tested conductivity, TDS, turbidity, or liquid level is greater than a preset value, or when a water column is tested, the analysis device (40) analyzes the test paper (203).

3. The toilet with the test member for urine test according to claim 1, wherein in an initial state, the support (202) retracts, and during testing, the support (202) is driven to extend to a preset area; the control unit further comprises a trapped water test circuit; the trapped water test circuit controls the sensing device (30) at the beginning of test to test the conductivity, TDS, turbidity, or liquid level of the trapped water; and when the conductivity, TDS, turbidity, or liquid level is less than the preset value, the control unit controls the support (202) to extend to the preset area.

4. The toilet with the test member for urine test according to claim 1, wherein the test member further comprises an outer shell (201), the outer shell (201) is provided with a first opening (201a) allowing the support (202) to extend and retract relative to the outer shell (201), and the support (202) is movable between an extended position relative to the outer shell (201) and a retracted position relative to the outer shell (201).

5. The toilet with the test member for urine test according to claim 4, wherein the outer shell (201) further comprises a second opening (201b) opposite to the first opening (201a), the support (202) is provided with a driving portion (204) at the end close to the second opening (201b), and when the support is at the retracted position, the driving portion (204) is located outside the outer shell (201).

6. The toilet with the test member for urine test according to claim 5, wherein the driving portion (204) comprises a rack (204a) and a motor (204b), the motor (204b) drives the rack (204a), and the rack (204a) is connected to the support (202) at the end close to the second opening (201b).

7. The toilet with the test member for urine test according to claim 6, wherein a sealing set is provided at two ends of the support (202); the sealing set comprises a first sealing member (202a) and a second sealing member (202b); when the support is located at the retracted position, the first sealing member (202a) is close to the first opening (201a), the second sealing member (202b) is close to the second opening (201b), and the support (202) and the outer shell (201) are sealed by the first sealing member (202a) and the second sealing member (202b) to form a sealing chamber for accommodating the test paper (203); and when the support is located at the extended position, the second sealing member (202b) is close to the first opening (201a).

8. The toilet with the test member for urine test according to claim 7, wherein the analysis device (40) is a color sensor, and during analysis, the support (202) retracts from the second opening (201b) for test and analysis.

9. The toilet with the test member for urine test according to claim 1, wherein the sensing device (30) is a conductivity sensor, a turbidity sensor, a liquid level sensor, or a radar, and the sensing device (30) is arranged diagonally upward from a horizontal line.

10. The toilet with the test member for urine test according to claim 1, wherein the test member (20) is installed at the toilet body; in the initial state, the support (202) retracts; and during testing, the support (202) is driven to extend into the bowl of the toilet body (10).

11. The toilet with the test member for urine test according to claim 10, wherein the sensing device (30) tests the conductivity or TDS of the trapped water in the bowl of the toilet body (10), the turbidity of the trapped water, the liquid level of the trapped water, or whether there is a water column in the bowl; and after the tested conductivity value, TDS value, turbidity value, or liquid level value is greater than a preset value, or after the variation value of the tested conductivity, TDS, turbidity, or liquid level is greater than a preset value, or when a water column is tested, the control unit controls the support (202) to retract, and the analysis device (40) analyzes the test paper (203).
